# EUROPEAN PATENT APPLICATION

(11) **EP 3 878 509 A1**
(43) Date of publication of application: **15.09.2021**
(21) Application number: 19881228.1
(22) Date of filing: 31.05.2019
(51) Int. Cl.: A61N 5/10

(54) **ELECTRONIC RADIOTHERAPY APPARATUS AND ELECTRON BEAM TRANSMISSION STRUCTURE APPLIED TO APPARATUS, AND BEAM NEEDLE**

(30) Priority: 05.11.2018 CN 201811308102
(71) Applicant: Shenzhen Mingjie Medical Technology Co. Ltd., Shenzhen, Guangdong 518048 (CN)
(72) Inventor: GAI, Wei, ShenZhen, Guangdong 518048 (CN); WANG, Ping, Shenzhen, Guangdong 518048 (CN)
(74) Representative: Boult Wade Tennant LLP
(86) International application number: PCT/CN2019/089580
(87) International publication number: WO 2020/093690

(57) **Abstract**

The present invention relates to the technical field of medical apparatuses, and specifically discloses a radiotherapy apparatus, and an electron beam needle and an electron beam transmission structure thereof. The beam needle comprises a needle body and an exit sealing sheet, wherein the needle body comprises a first end port and a second end port, and the first end port is in communication with the second end port; the exit sealing sheet is arranged at the second end port and seals the second end port; and when the beam needle is in a working state, the interior of the beam needle is in a vacuum state, and electrons enter the needle body from the first end port and are emitted through the exit sealing sheet from the second end port. The present invention provides a beam needle, an electron beam transmission structure of a radiotherapy apparatus, and a radiotherapy apparatus. The beam needle enters a body at a position corresponding to an area where a tumor is located. When the beam needle is in a working state, the interior thereof is in a vacuum state so as to reduce the attenuation of electrons and reduce the damage to normal tissues surrounding a tumor lesion site.

## Description

The present application claims priority to Chinese Patent Application No. CN201811308102.4, titled "CONNECTION STRUCTURE BETWEEN ACCELERATING TUBE AND BEAM NEEDLE, AND RADIOTHERAPY APPARATUS", filed on November 5, 2018 with the China National Intellectual Property Administration, which is incorporated herein by reference in its entirety.

### FIELD

The present disclosure generally relates to the technical field of medical apparatus, and in particular to a beam needle and an electron beam transmission structure applied to an electronic radiotherapy apparatus, and an electronic radiotherapy apparatus including the electron beam transmission structure.

### BACKGROUND

Tumour is a disease endangering human life. A tumor therapy method mainly depends on surgery, chemotherapy and radiotherapy. However, the surgery, the chemotherapy and the radiotherapy all cause deep pain to a patient. For example, the surgery not only has high treatment cost, but also has high risk and great therapy difficulty. In addition, the patient is required to go through a long period of wound healing and convalescence after surgery. X-rays have strong penetrability. In therapy, the X-rays are irradiated from outside the body of the patient and pass through the body. Therefore, a lot of energy is accumulated in normal tissues in front of and behind a tumour lesion site, which causes harm to the body.

### SUMMARY

An object of the present disclosure is to provide a radiotherapy apparatus, a beam needle and an electron beam transmission structure applied to the radiotherapy apparatus, to reduce harm to normal tissues around a tumor lesion site.

In order to achieve the above object, an electron beam needle of a radiotherapy apparatus is provided according to the disclosure. The beam needle is configured to enter an area where a tumor is located in a body. The beam needle includes a needle body. The needle body includes a first port and a second port. The first port is communicated with the second port. An outlet sealing sheet is arranged at the second port. The outlet sealing sheet is configured to seal the second port. An inner space of the beam needle is in a vacuum state when the beam needle is in an operating state. Electrons enter the needle body through the first port and are shot out from the needle body through the outlet sealing sheet at the second port.

In an embodiment, a thickness of the outlet sealing sheet is less than 200µm.

In an embodiment, the beam needle further includes a connection portion. The connection portion is provided with a through hole configured to transmit an electron beam, and the through hole is communicated with the first port.

In an embodiment, the beam needle is formed integrally.

In an embodiment, the outlet sealing sheet is connected to the needle body through welding.

In an embodiment, the outlet sealing sheet is made of stainless steel, titanium or beryllium.

Another electron beam needle of a radiotherapy apparatus is provided according to the disclosure. The beam needle is configured to enter an area where a tumor is located in a body. The electron beam needle is hollow inside. Two ports of the beam needle are sealed. The beam needle includes an inlet sealed port and an outlet sealed port. An internal space of the beam needle is in a vacuum state when the beam needle is in an operating state. Electrons enter the beam needle by passing through the inlet sealed port and are shot out from the beam needle by passing through the outlet sealed port.

In an embodiment, a thickness of each of the inlet sealed port and the outlet sealed port is less than 200µm.

In an embodiment, the beam needle includes a connection portion and a needle body. The needle body is connected to the connection portion. The inlet sealed port is arranged at the connection portion, and the outlet sealed port is arranged at an outlet of the needle body.

In an embodiment, the beam needle is formed integrally, or the connection portion is connected to the needle body through welding.

In an embodiment, each of the inlet sealed port and the outlet sealed port is made of stainless steel, titanium or beryllium.

An electron beam transmission structure of a radiotherapy apparatus is provided according to the disclosure. The electron beam transmission structure includes an accelerating tube and a beam needle. A first opening is arranged at an end of the accelerating tube. The beam needle includes a needle body. The needle body includes a first port and a second port. The first port is communicated with the first opening of the accelerating tube. The second port is communicated with the first port. An outlet sealing sheet is arranged at the second port of the needle body, and the outlet sealing sheet is configured to seal the second port. An inner space the electron beam transmission structure in which the accelerating tube is communicated with the beam needle and through which an electron beam passes is in a vacuum state when the electron beam transmission structure is in an operating state. Electrons, shot out from the accelerating tube, enter the needle body through the first port of the beam needle and then are shot out from the needle body by passing through the outlet sealing sheet at the second port.

In an embodiment, a thickness of the outlet sealing sheet is less than 200µm.

In an embodiment, the beam needle includes a connection portion arranged at the first port, and the connection portion is in fixed connection with the accelerating tube.

Another electron beam transmission structure of a radiotherapy apparatus is provided according to the disclosure. The electron beam transmission structure includes an accelerating tube and a beam needle. A first opening is arranged at an end of the accelerating tube, and a sealing sheet is arranged at the first opening. The electron beam needle is hollow inside. Two ports of the beam needle are sealed. The beam needle includes an inlet sealed port and an outlet sealed port. An inner space of the electron beam transmission structure in which the accelerating tube is communicated with the beam needle and through which an electron beam passes is in a vacuum state when the electron beam transmission structure is in an operating state. Electrons, shot out from the accelerating tube through the sealing sheet, enter the beam needle through the inlet sealed port of the beam needle and are shot out from the beam needle by passing through the outlet sealed port of the beam needle.

In an embodiment, a thickness of the outlet sealed port of the needle body is less than 200µm.

In an embodiment, the beam needle includes a connection portion and a needle body. The connection portion is in fixed connection with the accelerating tube. The inlet sealed port is arranged at the connection portion and corresponds to the sealing sheet of the accelerating tube. The outlet sealed port is arranged at an outlet of the needle body.

In an embodiment, the electron beam transmission structure further includes a focusing coil configured to focus electrons, that are scattered after passing through the sealing sheet of the accelerating tube and the inlet sealed port of the beam needle, on an axis of the beam needle.

An electronic radiotherapy apparatus is provided according to the disclosure. The electronic radiotherapy apparatus includes the electron beam transmission structure of a radiotherapy apparatus described above.

An advantageous effect of the present disclosure is to provide a beam needle, an electron beam transmission structure of a radiotherapy apparatus, and a radiotherapy apparatus, to realize electron radiation therapy in a body, thereby reducing harm to normal tissues. The beam needle is provided with an outlet sealing sheet, so that a space through which an electron beam passes is in a vacuum state, thereby reducing electron attenuation.

### BRIEF DESCRIPTION OF THE DRAWINGS

In order to more clearly describe the technical solutions in the embodiments of the present disclosure or the technical solutions in the conventional technology, drawings to be used in the description of the embodiments of the present disclosure or the conventional technology are briefly described hereinafter. It is apparent that the drawings described below show merely the embodiments of the present disclosure, and those skilled in the art may obtain other drawings according to the provided drawings without any creative effort.
Figure 1 is a schematic structural diagram of a beam needle according to a first embodiment of the present disclosure;
Figure 2 is a schematic structural diagram of the beam needle according to the first embodiment of the present disclosure viewed from another point of view;
Figure 3 is a schematic diagram of an electron beam transmission structure of a radiotherapy apparatus according to the first embodiment of the present disclosure; and
Figure 4 is a schematic diagram of an electron beam transmission structure of a radiotherapy apparatus according to a second embodiment of the present disclosure.

In the drawings:

| | |
|---|---|
| 1 Accelerating tube | 11 First opening |
| 2 First titanium sheet | 3 Connection portion |
| 4 Second titanium sheet | 5 Beam needle |
| 51 Needle body | 52 First port |
| 53 Second port | 6 Block sheet |
| 7 Focusing coil | 8 Flange |
| 100 Electron beam transmission structure | 200 Electron beam transmission structure |

### DETAILED DESCRIPTION OF EMBODIMENTS

In order to make the objective, features and advantages of the present disclosure obvious and easy to be understood, technical solutions of embodiments of the present disclosure are clearly and completely described below in conjunction with the drawings of the embodiments of the present disclosure. Apparently, the embodiments described in the following are only some embodiments of the present disclosure, rather than all the embodiments. Any other embodiments obtained by those skilled in the art based on the embodiments in the present disclosure without any creative effort fall within the protection scope of the present disclosure.

In the descriptions of the present disclosure, it should be understood that in a case that a module is regarded to be "connected to" another module, the module may be directly connected to the another module or connected to the another module through a third module arranged between the module and the another module. In a case that a module is regarded to be "arranged on" another module, the module may be directly arranged on the another module or arranged on the another module through a third module arranged between the module and the another module.

In addition, an orientation or a position relation indicated by terms such as "long", "short", "inside" and "outside" is based on an orientation or a position relation shown in the drawings, which is only for convenience of the descriptions of the present disclosure, rather than indicating or implying that a device or an element referred to must have a particular orientation or an operation is performed based on a particular orientation. Therefore, the terms indicating an orientation or a position relation should not be understood as a limitation of the present disclosure.

Technical solutions of the present disclosure are described based on embodiments in conjunction with the drawings.

A radiotherapy apparatus according to the present disclosure is an electronic radiotherapy apparatus. The electronic radiotherapy apparatus includes an electron beam transmission structure. The electron beam transmission structure includes a beam needle. The beam needle is configured to enter a body to cause an electron beam emitted by the electronic radiotherapy apparatus to be shot into the body through the beam needle, so as to ablate a diseased tissue, for example, a tumour.

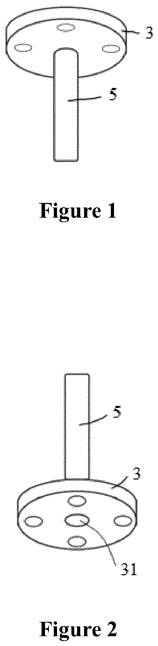

Figure 1 and Figure 2 are diagrams showing a beam needle according to a first embodiment of the present disclosure respectively viewed from different points of view. The beam needle 5 includes a connection portion 3, a needle body 51 and an outlet sealing sheet 6. The connection portion 3 is provided with a through hole 31 along a central axis of the connection portion 3. The through hole 31 is configured to transmit an electron beam. The needle body 51 includes a first port 52 and a second port 53. The second port 53 is communicated with the first port 52. The through hole of the connection portion 3 is communicated with the first port 52. In this way, an internal hollow channel is formed in the beam needle 5 for an electron beam to pass through. The outlet sealing sheet 6 is arranged at the second port 53. The outlet sealing sheet 6 seals the second port 53. An internal space of the beam needle 5 is in a vacuum state when the beam needle 5 is in an operating state. Electrons enter the needle body 51 through the first port 52, and are shot out from the needle body 51 by passing through the outlet sealing sheet 6 at the second port 53.

The connection portion 3 may be formed integrally with the needle body 51. Alternatively, the connection portion 3 may be connected to the needle body 51 through welding. The connection portion 3 and the needle body 51 may be made of the same material or different materials.

In the embodiment, in order to ensure that an internal space of the beam needle 5 is in a vacuum state, a needle opening of the beam needle 5, that is, the second port 53, is sealed by the outlet sealing sheet 6. The outlet sealing sheet 6 may be formed integrally with the beam needle 5 (in this case, the outlet sealing sheet 6 is integrated with the beam needle 5). Based on conventional technologies, a wall of the needle body of the beam needle 5 may be thick enough and a thickness of the outlet sealing sheet 6 may be small enough, so that the electrons can be shot out only from the outlet sealing sheet 6. In addition, in order to reduce attenuation of the electrons, a thickness of the outlet sealing sheet 6 may be less than 200µm. The outlet sealing sheet 6 may be made of titanium (in this case, the outlet sealing sheet 6 is not formed integrally with the beam needle 5). It can be understood that an inclination angle of the outlet sealing sheet 6 relative to the needle body 51 of the beam needle 5 may be adjusted according to actual needs. The inclination angle ranges from 0 degree to 180 degree. Preferably, the outlet sealing sheet 6 is perpendicular to a central axis of the needle body 51.

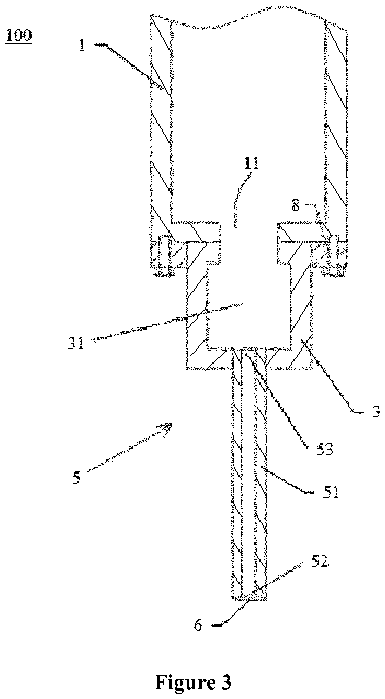

Figure 3 is a schematic diagram of an electron beam transmission structure according to an embodiment of the present disclosure. The electron beam transmission structure 100 includes an accelerating tube 1 and a beam needle 5. A connection portion 3 of the beam needle 5 is fixed on the accelerating tube 1. The beam needle 5 is configured to enter an area where a tumor is located in a body.

A first opening 11 is arranged at a bottom of the accelerating tube 1. The first opening 11 is communicated with a through hole 31 of the beam needle 5, for an electron beam to pass.

The accelerating tube 1 may be connected to the connection portion 3 of the beam needle 5 through a flange 8 or a thread. The connection portion 3 may be in sealed connection with the beam needle 5 through welding or a taper thread.

It can be understood that the beam needle 5 may be fixed on the accelerating tube 1 through connection. Alternatively, the beam needle 5 may be formed integrally with the accelerating tube 1. The beam needle 5 may be fixed on the accelerating tube 1 through welding or a bolt.

In therapy, the beam needle 5 is inserted directly into a body of a patient or drawn into the body of the patient through a trocar. An electron beam enters the beam needle by passing through the first opening 11 of the accelerating tube 1 and then is shot out from the beam needle through an outlet sealing sheet, so as to ablate a diseased tissue, for example, a tumour, in the body of the patient.

In the embodiment, the outlet sealing sheet 6 is arranged at the second port 53 and seals the second port 53. An internal space of beam needle is in a vacuum state when the beam needle is in an operating state, thereby reducing electron attenuation and reducing harm to normal tissues around a tumour lesion site.

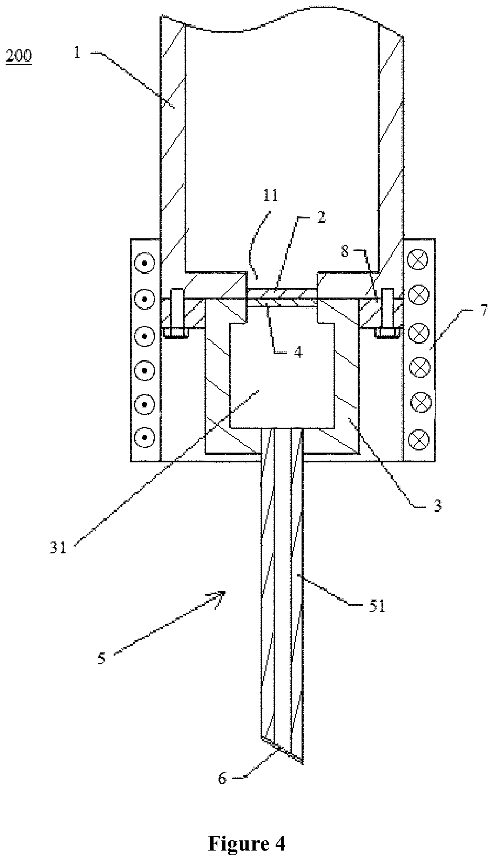

An electron beam needle of a radiotherapy apparatus is provided according to another embodiment of the present disclosure, referring to Figure 4, a beam needle 5 includes a connection portion 3 and a needle body 51. The needle body 51 is connected to the connection portion 3. The beam needle 5 may be formed integrally. Alternatively, the connection portion 3 is connected to the needle body 51 through welding. The beam needle 5 is hollow inside. Two ports of the beam needle 5 are sealed. The beam needle 5 includes an inlet sealed port 4 and an outlet sealed port 6. The inlet sealed port 4 is arranged at the connection portion 3. The outlet sealed port 6 is arranged at an outlet of the needle body 51. An internal space of beam needle 5 is in a vacuum state when the beam needle 5 is in an operating state. Electrons enter the beam needle 5 by passing through the inlet sealed port 4 and are shot out from the beam needle 5 through the outlet sealed port 6.

Preferably, a thickness of the outlet sealed port 6 is less than 200µm.

The outlet sealed port may be made of any material through which electrons can pass. The material is preferably stainless steel, titanium or beryllium.

Figure 4 shows a connection structure 200 through which an accelerating tube is connected to a beam needle according to another embodiment of the present disclosure. A first opening 11 is arranged at an end of the accelerating tube 1. The first opening window 11 is provided with a sealing sheet, that is, a first titanium sheet 2. A second titanium sheet 4 is arranged at the inlet sealed port of the connection portion 3 close to the accelerating tube 1. An outlet sealing sheet 6 is arranged at the outlet sealed port of the needle body 51 of the beam needle 5. A port at an end of the beam needle 5 is communicated with a through hole 31 of the connection portion 3. A port at another end of the beam needle extends to outside of the connection portion 3 and is sealed by the outlet sealing sheet 6.

An inner space of the accelerating tube 1 is sealed by the first titanium sheet 2. An inner space of the connection portion 3 is sealed by the second titanium sheet 4. A needle opening of the beam needle 5 is sealed by the outlet sealing sheet 6. Therefore, when the accelerating tube 1 is connected to the connection portion 3 or separated from the connection portion 3, vacuum states respectively in the accelerating tube 1 and the beam needle 5 are not be damaged. With the radiotherapy apparatus and the connection structure through which the accelerating tube 1 is connected to the beam needle 5 according to the embodiment, the beam needle 5 and the connection portion 3 are required to be replaced as a whole when the beam needle 5 is required to be replaced.

In the embodiment, the connection structure through which the accelerating tube 1 is connected to the beam needle 5 further includes a focusing coil 7 configured to focus electrons in the connection potion 3 on an axis of the beam needle 5. Electrons from the accelerating tube 1 may scatter in the connection portion 3 after passing through the first titanium sheet 2, a gap between the first titanium sheet 2 and the second titanium sheet 4, and the second titanium sheet 4. The focusing coil 7 can focus the scattered electrons in the beam needle 5, which helps to effectively guide a motion path of the electrons. A transmission path of the electrons is described as follows. The electrons enter the connection portion 3 from the accelerating tube 1 after passing through the first titanium sheet 2 and the second titanium sheet 4. Then the electrons passes through the needle body 51 of the beam needle 5 under an action of the focusing coil 7. Finally the electrons reach an area where a tumour is located after passing through the outlet sealing sheet 6.

In addition, a thickness of each of the first titanium sheet 2 and the second titanium sheet 4 is less than 30µm (preferably less than 200µm). A distance between a surface of the first titanium sheet 2 away from the second titanium sheet 4 and a surface of the second titanium sheet 4 away from the first titanium sheet 2 is less than 60µm (preferably less than 50µm). Walls of the accelerating tube 1 and the connection portion 3 are thick enough so as to avoid electrons from passing through the walls. Long-term experiments and researches indicate that transmission of electrons is better in a case that titanium is used as a sealing material. Especially, in a case that a thickness of each of the first titanium sheet 2 and the second titanium sheet 4 is less than 30µm or a distance between a surface of the first titanium sheet 2 away from the second titanium sheet 4 and a surface of the second titanium sheet 4 away from the first titanium sheet 2 is less than 60µm, attenuation of electrons is light. In this case, both an effective electron pass rate and seal of a space inside the accelerating tube can be ensured.

In the embodiment, in order to ensure that an internal space of the through hole 3 of the connection portion 3 is in a vacuum state, the second port 53 of the beam needle 5 is sealed by the outlet sealing sheet 6. The outlet sealing sheet 6 may be formed integrally with the needle body 51 of the beam needle 5 (in this case, the outlet sealing sheet 6 is integrated with the beam needle 5). Based on conventional technologies, a wall of the beam needle 5 may be thick enough and a thickness of the outlet sealing sheet 6 may be small enough, so that the electrons can be shot out only from the outlet sealing sheet 6. In addition, in order to reduce attenuation of the electrons, a thickness of the outlet sealing sheet 6 may be less than 200µm. The outlet sealing sheet 6 may be made of titanium (in this case, the outlet sealing sheet 6 is not formed integrally with the beam needle 5). The outlet sealing sheet 6 may be preferably made of stainless steel, titanium or beryllium. Alternatively, the outlet sealing sheet 6 may be made of other materials that can be processed into a sheet with a small thickness.

In the embodiment, the accelerating tube 1 may be connected to the connection portion 3 through a flange 8 or a thread. The connection portion 3 may be in sealed connection with the beam needle 5 through welding or a taper thread.

It can be understood that the beam needle 5 may be fixed on the accelerating tube 1 through welding or a bolt. Alternatively, the beam needle 5 may be formed integrally with the accelerating tube 1.

The above embodiments are only used to explain the technical solutions of the present disclosure rather than limit the technical solutions of the present disclosure. Though the present disclosure is described in detail in conjunction with the above embodiments, those skilled in the art should understand that modifications may be made based on the technical solutions in the above embodiments and equivalent substitutions may be made to partial technical features of the technical solutions. Such modifications or substitutions do not cause nature of corresponding technical solutions deviate the spirit and the scope of the technical solutions in the embodiments of the present disclosure.

## Claims

1. An electron beam needle of a radiotherapy apparatus, the beam needle being configured to enter an area where a tumor is located in a body, the beam needle comprising:
a needle body comprising a first port and a second port, wherein the first port is communicated with the second port, an outlet sealing sheet is arranged at the second port, and the outlet sealing sheet is configured to seal the second port; and wherein
an inner space of the beam needle is in a vacuum state when the beam needle is in an operating state, electrons enter the needle body through the first port and are shot out from the needle body through the outlet sealing sheet at the second port.

2. The beam needle according to claim 1, wherein a thickness of the outlet sealing sheet is less than 200µm.

3. The beam needle according to claim 1, further comprising a connection portion, wherein the connection portion is provided with a through hole configured to transmit an electron beam, and the through hole is communicated with the first port.

4. The beam needle according to claim 1, wherein the beam needle is formed integrally.

5. The beam needle according to claim 1, wherein the outlet sealing sheet is connected to the needle body through welding.

6. The beam needle according to claim 1, wherein the outlet sealing sheet is made of stainless steel, titanium or beryllium.

7. An electron beam needle of a radiotherapy apparatus, the beam needle being configured to enter an area where a tumor is located in a body, the electron beam needle being hollow inside, wherein two ports of the beam needle are sealed, the beam needle comprises an inlet sealed port and an outlet sealed port, an internal space of the beam needle is in a vacuum state when the beam needle is in an operating state, electrons enter the beam needle by passing through the inlet sealed port and are shot out from the beam needle by passing through the outlet sealed port.

8. The beam needle according to claim 7, wherein a thickness of each of the inlet sealed port and the outlet sealed port is less than 200µm.

9. The beam needle according to claim 7, comprising a connection portion and a needle body, wherein the needle body is connected to the connection portion, the inlet sealed port is arranged at the connection portion, and the outlet sealed port is arranged at an outlet of the needle body.

10. The beam needle according to claim 9, wherein the beam needle is formed integrally or the connection portion is connected to the needle body through welding.

11. The beam needle according to claim 1, wherein each of the inlet sealed port and the outlet sealed port is made of stainless steel, titanium or beryllium.

12. An electron beam transmission structure of a radiotherapy apparatus, the electron beam transmission structure comprising:
an accelerating tube, wherein a first opening is arranged at an end of the accelerating tube; and
a beam needle comprising a needle body, wherein the needle body comprises a first port and a second port, wherein the first port is communicated with the first opening of the accelerating tube, the second port is communicated with the first port, an outlet sealing sheet is arranged at the second port of the needle body, and the outlet sealing sheet is configured to seal the second port, wherein,
an inner space of the electron beam transmission structure in which the accelerating tube is communicated with the beam needle and through which an electron beam passes is in a vacuum state when the electron beam transmission structure is in an operating state, wherein electrons, shot out from the accelerating tube, enter the needle body through the first port of the beam needle and are shot out from the needle body by passing through the outlet sealing sheet at the second port.

13. The electron beam transmission structure according to claim 12, wherein a thickness of the outlet sealing sheet is less than 200µm.

14. The electron beam transmission structure according to claim 12, wherein the beam needle comprises a connection portion arranged at the first port, and the connection portion is in fixed connection with the accelerating tube.

15. An electron beam transmission structure of a radiotherapy apparatus, the electron beam transmission structure comprising
an accelerating tube, wherein a first opening is arranged at an end of the accelerating tube, and a sealing sheet is arranged at the first opening; and
a beam needle, wherein the electron beam needle is hollow inside, two ports of the beam needle are sealed, the beam needle comprises an inlet sealed port and an outlet sealed port, wherein
an inner space of the electron beam transmission structure in which the accelerating tube is communicated with the beam needle and through which an electron beam passes is in a vacuum state when the electron beam transmission structure is in an operating state, wherein electrons, shot out from the accelerating tube through the sealing sheet, enter the beam needle through the inlet sealed port of the beam needle and are shot out from the beam needle by passing through the outlet sealed port of the beam needle.

16. The electron beam transmission structure according to claim 15, wherein a thickness of the outlet sealed port of the needle body is less than 200µm.

17. The electron beam transmission structure according to claim 15, wherein
the beam needle comprises a connection portion and a needle body, wherein the connection portion is in fixed connection with the accelerating tube;
the inlet sealed port is arranged at the connection portion and corresponds to the sealing sheet of the accelerating tube; and
the outlet sealed port is arranged at an outlet of the needle body.

18. The electron beam transmission structure according to claim 15, further comprising a focusing coil configured to focus electrons, that are scattered after passing through the sealing sheet of the accelerating tube and the inlet sealed port of the beam needle, on an axis of the beam needle.

19. An electronic radiotherapy apparatus comprising the electron beam transmission structure of a radiotherapy apparatus according to any one of claims 12 to 18.
